Europäisches Patentamt

European Patent Office

Office européen des brevets

(11) Veröffentlichungsnummer: **0 360 092**

**A1**

# (12) EUROPÄISCHE PATENTANMELDUNG

(21) Anmeldenummer: 89116612.6

(22) Anmeldetag: 08.09.89

(51) Int. Cl.⁵: **C07C 37/04 , C07C 39/15 , C07C 303/06 , C07C 303/08**

(30) Priorität: 17.09.88 DE 3831712

(43) Veröffentlichungstag der Anmeldung:
28.03.90 Patentblatt 90/13

(84) Benannte Vertragsstaaten:
CH DE FR GB IT LI

(71) Anmelder: **BASF Aktiengesellschaft**
**Carl-Bosch-Strasse 38**
**D-6700 Ludwigshafen(DE)**

(72) Erfinder: **Eichenauer, Ulrich, Dr.**
**Paul-Ehrlich-Strasse 25 a**
**D-6000 Frankfurt 70(DE)**

(54) Verfahren zur Herstellung von 4-4"-Dihydroxyterphenyl.

(57) Verfahren zur Herstellung von 4,4"-Dihydroxyterphenyl durch Sulfonierung von Terphenyl mit Schwefelsäure, Schwefeltrioxid oder deren Mischung oder mit Chlorsulfonsäure und anschließender Behandlung der resultierenden Terphenyl-4,4"-disulfonsäure, gegebenenfalls in Form ihrer Dialkalisalze, mit Alkalihydroxid.

EP 0 360 092 A1

## Verfahren zur Herstellung von 4,4″-Dihydroxyterphenyl

Die vorliegende Erfindung betrifft ein neues Verfahren zur Herstellung von 4,4″-Dihydroxyterphenyl durch Sulfonierung von Terphenyl und anschließender Behandlung der resultierenden Terphenyl-4,4″-disulfonsäure, gegebenenfalls in Form ihrer Dialkalisalze, mit Alkalihydroxid.

Aus J. Am. Chem. Soc. 66, 632 (1944) ist es bekannt, Terphenyl (1,4-Diphenylbenzol) zu nitrieren, die resultierende Dinitroverbindung dann zu reduzieren und das entstehende 4,4″-Diaminoterphenyl schließlich zu tetrazotieren und daran anschließend zu verkochen.

Diese Herstellmethode für 4,4″-Dihydroxyterphenyl ist jedoch mehrstufig, wobei insbesondere der letzte Schritt nur in unbefriedigender Ausbeute abläuft. Außerdem entstehen beim Verfahren des Standes der Technik unerwünschte und nur schwer abtrennbare Positionsisomere.

Aufgabe der vorliegenden Erfindung war es deshalb, ein neues Verfahren bereitzustellen, mittels dessen die Herstellung von 4,4″-Dihydroxyterphenyl in einfacher Weise gelingen und das Zielprodukt in guter Ausbeute und hoher Reinheit anfallen sollte.

Es wurde nun gefunden, daß die Herstellung von 4,4″-Dihydroxyterphenyl vorteilhaft gelingt, wenn man Terphenyl mit Schwefelsäure, Schwefeltrioxid oder deren Mischung oder mit Chlorsulfonsäure sulfoniert, die resultierende Terphenyl-4,4″-disulfonsäure gegebenenfalls in ihre Dialkalisalze überführt, und sie anschließend mit Alkalihydroxid behandelt.

Die Sulfonierung von Terphenyl erfolgt erfindungsgemäß mit Schwefelsäure, mit Schwefeltrioxid, mit Oleum oder mit Chlorsulfonsäure. Verwendet man Schwefelsäure als Sulfonierungsreagenz, so sollte diese eine Konzentration von 85 Gew.% oder darüber aufweisen. Oleum sollte im allgemeinen einen Gehalt von 5 bis 65 Gew.% an Schwefeltrioxid aufweisen.

Die Verwendung von Schwefelsäure als Sulfonierungsreagenz ist bevorzugt.

Als Reaktionsmedium für die Sulfonierung mit Schwefeltrioxid können flüssiges Schwefeldioxid oder inerte organische Lösungsmittel, wie halogenierte Kohlenwasserstoffe oder halogenierte Aromaten, z.B. Methylenchlorid, Chloroform, Tetrachlorkohlenstoff, Dichlorbenzol, Trichlorbenzol oder Brombenzol, verwendet werden. Bei Verwendung von Chlorsulfonsäure, Schwefelsäure oder Oleum als Sulfonierungsreagenz können diese Reaktionspartner gleichzeitig auch als Reaktionsmedium verwendet werden.

Die Sulfonierung wird im allgemeinen bei einer Temperatur von 0 bis 140°C, bei Verwendung von Schwefelsäure vorzugsweise von 100 bis 140°C und bei Verwendung von Schwefeltrioxid in inerten Lösungsmitteln vorzugsweise von 0 bis 40°C, durchgeführt. Die Menge des Sulfonierungsmittels ist nicht kritisch. Dies bedeutet, es kann molar (2 Mol Sulfonierungsmittel je Mol Terphenyl) oder auch im Überschuß verwendet werden.

Zweckmäßig wird die Sulfonierung so vorgenommen, daß man Sulfonierungsreagenz und gegebenenfalls inertes Lösungsmittel vorlegt, dazu dann Terphenyl gibt und dieses Gemisch anschließend, gegebenenfalls unter Erwärmen, bei der obengenannten Temperatur rührt.

Nach einer Reaktionsdauer von ca. 2 bis 6 Stunden wird das Reaktionsgemisch mit Wasser verdünnt, wobei sich die gebildete Terphenyl-4,4″-disulfonsäure als Feststoff abscheidet und gegebenenfalls nach Abtrennung des inerten Lösungsmittels durch Filtration isoliert werden kann.

Die Terphenyl-4,4″-disulfonsäure kann nun entweder in freier Form oder in Form ihrer Dialkalisalze der Behandlung mit Alkalihydroxid unterworfen werden.

Wenn man die Disulfonsäure in Form ihrer Dialkalisalze, insbesondere der Natrium- oder Kaliumsalze, für die Weiterreaktion verwendet, so können diese Dialkalisalze nach an sich bekannten Methoden erhalten werden, beispielsweise durch Aussalzen einer wäßrigen Lösung oder Suspension der Disulfonsäure mit Alkalisalzen, z.B. mit Natrium- oder Kaliumchlorid, oder Neutralisation einer wäßrigen Lösung oder Suspension der Disulfonsäure mit Alkalihydroxiden, z.B. mit Natrium- oder Kaliumhydroxid.

Zur Substitution der Sulfonsäuregruppen durch den Hydroxylrest werden die Terphenyl-4,4″-disulfonsäure oder ihre Dialkalisalze mit Alkalihydroxid behandelt.

Die Behandlung erfolgt entweder mit wäßrigem Alkalihydroxid bei einer Temperatur von 200 bis 360°C, vorzugsweise 300 bis 340°C, unter dem sich einstellenden Druck oder vorzugsweise in einer Alkalihydroxid-Schmelze bei einer Temperatur von 200 bis 360°C, vorzugsweise 300 bis 340°C.

Beim Arbeiten mit wäßrigem Alkalihydroxid verwendet man im allgemeinen eine wäßrige Lösung, die 20 bis 70 Gew.% Alkalihydroxid enthält. Geeignete Alkalihydroxide sind z.B. Natriumhydroxid, Kaliumhydroxid oder deren Mischungen. Bevorzugt ist eine Mischung, die bis zu 25 Gew.% Natriumhydroxid enthält. Ganz besonders hervorzuheben ist die Verwendung von Kaliumhydroxid.

In manchen Fällen empfiehlt es sich, ein Alkalicarbonat, z.B. Natrium- oder Kaliumcarbonat als Schaumverhinderer hinzuzugeben. In der Regel werden dazu 5 bis 20 Gew.%, an Alkalicarbonat, bezogen auf das Alkalihydroxid, zugegeben. Die Menge an Alkalihydroxid beträgt im allgemeinen 200 bis 400 Gew.% bezogen auf eingesetztes Substrat.

Zweckmäßig erfolgt die Behandlung mit Alkalihydroxid so, daß man Alkalihydroxid und gegebenenfalls Alkalicarbonat als wäßrige Lösung oder in Substanz vorlegt, anschließend auf die obengenannte Temperatur erhitzt, wobei sich im letzteren Fall eine Schmelze ausbildet, dann die Terphenyl-4,4″-disulfonsäure oder ihre Dialkalisalze zugibt und ca. 2 bis 4 Stunden bei der obengenannten Temperatur hält, wobei im Falle der wäßrigen Alkalihydroxidlösung der sich einstellende Druck einzuhalten ist.

Nach beendeter Umsetzung wird das Reaktionsgemisch abgekühlt, was im Falle der Schmelze durch kontrollierte Wasserzugabe erfolgen kann, mit Wasser weiter verdünnt und angesäuert, wobei sich 4,4″-Dihydroxyterphenyl abscheidet.

Nach dem Abtrennen des Zielprodukts kann dies durch Umkristallisieren aus hochsiedenden Carbonamiden, wie N,N-Dimethylformamid, N,N-Dimethylacetamid, N-Methylpyrrolidin-2-on, 1,3-Dimethylimidazolidin-2-on oder 1,3-Dimethylhexahydropyrimidin-2-on, weiter gereinigt werden.

Verglichen mit dem Verfahren des Standes der Technik ist das erfindungsgemäße Verfahren technisch wesentlich einfacher durchzuführen. Das Zielprodukt fällt dabei in hoher Ausbeute und Reinheit an.

Überraschenderweise erfolgt bei der Sulfonierung der Einbau der Sulfonsäuregruppen praktisch ausschließlich in der 4,4″-Position, obwohl die gleichzeitige Bildung von anderen Stellungsisomeren oder von höher sulfonierten Produkten zu erwarten gewesen wäre.

4,4″-Dihydroxyterphenyl ist ein wertvolles Monomer für die Herstellung von flüssigkristallinen Polyestern.

Das folgende Beispiel soll die Erfindung näher erläutern.

a) Terphenyl-4,4″-disulfonsäure

880 g konz. Schwefelsäure wurden auf 110° C erwärmt und darin 270,5 g Terphenyl eingetragen. Die Temperatur wurde auf 140° C gesteigert und 4 Stunden dort belassen. Nach Abkühlen auf 100° C wurden 800 ml Wasser zugetropft, dann ließ man das Gemisch erkalten. Nach dem Absaugen erhielt man ein Rohprodukt, das direkt in die nächste Stufe eingesetzt wurde. Reinheit (HPLC): 99,7 %; Ausbeute: 565 g.

b) 4,4″-Dihydroxyterphenyl

In einem 1 l-V₂A-Kessel mit Ankerrührer wurden 600 g Kaliumhydroxid und 60 g Natriumcarbonat vorgelegt und bei 300° C 250 g des unter a) beschriebenen Rohprodukts portionsweise innerhalb von 2 Stunden eingetragen. Die Temperatur wurde auf 330° C gesteigert und 3 Stunden nachgerührt. Durch Wasserzugabe wurde kontrolliert abgekühlt, mit insgesamt 1,6 l Wasser verdünnt und abgesaugt. Der Rückstand wurde in 2 l Wasser suspendiert, mit konz. Salzsäure angesäuert, auf 90° C erwärmt und heiß abgesaugt. Der Rückstand wurde getrocknet und aus N,N-Dimethylformamid umkristallisiert. Reinheit (HPLC): 99,8 %; Ausbeute: 102,0 g (74 % bezogen auf Terphenyl).

## Ansprüche

1. Verfahren zur Herstellung von 4,4″-Dihydroxyterphenyl, dadurch gekennzeichnet, daß man Terphenyl mit Schwefelsäure, Schwefeltrioxid oder deren Mischung oder mit Chlorsulfonsäure sulfoniert, die resultierende Terphenyl-4,4″-disulfonsäure gegebenenfalls in ihre Dialkalisalze überführt, und sie anschließend mit Alkalihydroxid behandelt.

2. Verfahren gemäß Anspruch 1, dadurch gekennzeichnet, daß man die Sulfonierung bei einer Temperatur von 0 bis 140° C vornimmt.

3. Verfahren gemäß Anspruch 1, dadurch gekennzeichnet, daß man die Behandlung mit Alkalihydroxid bei einer Temperatur von 200 bis 360° C vornimmt.

4. Verfahren gemäß Anspruch 1, dadurch gekennzeichnet, daß man die Behandlung mit Alkalihydroxid in einer Alkalihydroxid-Schmelze durchführt.

Europäisches
Patentamt

**EUROPÄISCHER RECHERCHENBERICHT**

Nummer der Anmeldung

EP 89 11 6612

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 092 772 (K.K. UENO)<br>* Ansprüche 1-2,5; Seite 2, Zeilen 7-30 *<br>--- | 1,3 | C 07 C 37/04<br>C 07 C 39/15<br>C 07 C 303/06<br>C 07 C 303/08 |
| Y | EP-A-0 085 883 (BAYER AG)<br>* Anspruch 1 *<br>--- | 1,3 | |
| A | JOURNAL OF CHROMATOGRAPHY, Band. 11, Nr. 3, 1963, Seiten 339-343; E. DENTI et al.: "Paper chromatography of polyphenyls qualitative separation of sulphonic acid derivatives of diphenyl and o-, m- and p-terphenyls"<br>* Insgesamt *<br>----- | | |
| | | | **RECHERCHIERTE SACHGEBIETE (Int. Cl.5)**<br><br>C 07 C 37/00<br>C 07 C 39/00<br>C 07 C 303/00 |

Der vorliegende Recherchenbericht wurde für alle Patentansprüche erstellt

| Recherchenort | Abschlußdatum der Recherche | Prüfer |
|---|---|---|
| DEN HAAG | 11-12-1989 | KLAG M.J. |